# EUROPÄISCHE PATENTSCHRIFT

(11) **EP 0 959 768 B1**
(45) Veröffentlichungstag und Bekanntmachung des Hinweises auf die Patenterteilung: **04.07.2001**
(21) Anmeldenummer: 96943068.5
(22) Anmeldetag: 10.12.1996
(51) Int. Cl.: A61B 5/11, A61B 5/042

(54) **ENDOTRACHEAL-TUBUS**
ENDOTRACHEAL TUBE
TUBE ENDOTRACHEAL

(30) Priorität: 22.12.1995 DE 29520326 U; 20.03.1996 DE 29605130 U
(43) Veröffentlichungstag der Anmeldung: 01.12.1999
(73) Patentinhaber: Lamadé, Wolfram, 69118 Heidelberg (DE); Meyding-Lamadé, Ute, 69118 Heidelberg (DE)
(72) Erfinder: Lamadé, Wolfram, 69118 Heidelberg (DE); Meyding-Lamadé, Ute, 69118 Heidelberg (DE)
(74) Vertreter: Möhring, Friedrich
(86) Internationale Anmeldenummer: EP9605504
(87) Internationale Veröffentlichungsnummer: WO9723163

(56) Entgegenhaltungen:
- EP-A- 0 438 863
- DE-U- 8 915 538
- GB-A- 2 294 642
- NL-A- 7 908 514
- THE LARYNGOSCOPE, Bd. 101, September 1991, Seiten 1024-1025, XP000670661 J.A. SERCARZ ET AL.: "Fabrication of a Custom Electrode Endotracheal Tube"
- OTOLARYNGOLOGY - HEAD AND NECK SURGERY, Bd. 100, 1989, Seiten 137-145, XP000670663, Dale Smith et al: "A device for the intraoperative identification of the recurrent laryngeal nerve in piglets."

## Beschreibung

Die vorliegende Erfindung betrifft einen Mehrballon-Endotrachealtubus, umfassend einen Schlauchabschnitt und mindestens zwei einander benachbart angeordnete, unabhängig voneinander expandierbare Ballons, wobei einem ersten, oberen, oberhalb eines zweiten, unteren Ballons angeordneten Ballon atraumatische Sensoren zur Prüfung der motorischen Kehlkopfnerven zugeordnet sind.

Die Schädigung des Nervus laryngeus recurrens (NLR) ist eine schwerwiegende Komplikation bei Halsoperationen. Der beidseitige Funktionsausfall stellt eine lebensbedrohliche Notfallsituation dar. Große Sammelstatistiken ergaben bei über 12.000 dokumentierten Strumaresektionen eine permanente Recurrenspareserate von 1,2% bzw. 5,2% abhängig davon, ob der Nerv während der Operation dargestellt (freigelegt) wurde oder nicht. Es herrscht damit überwiegend Einigkeit darin, daß der NLR während Schilddrüsenoperationen dargestellt werden sollte, um eine accidentelle Verletzung auszuschließen. Ein varianter Verlauf und die Feinheit seiner Struktur führen häufig zu Schwierigkeiten bei der Identifizierung des Nervs. Dies trifft insbesondere auf große Strumen, Rezidiveingriffe und Tumoroperationen zu. Hier liegt die Verletzungsrate des NLR bei bis zu 20%. Die Schädigung des Nervus laryngeus superior (NLS) wird selbst bei unkomplizierten Strumen mit bis zu 25% aller Patienten angegeben. In der Mehrzahl der Fälle von postoperativen Funktionsstörungen des NLR kann der Operateur eine Durchtrennung des Nerven sicher ausschließen. Die Ursache der Schädigungen liegt daher vor allem in einer Dehnung, Kompression oder Einknotung des Nerven in der chirurgischen Naht begründet. Hieraus ergibt sich die Dringlichkeit eines kontinuierlichen, intraoperativen Monitoring des NLR, um eine drohende Verletzung des Nerven bei der Mobilisierung der Schilddrüse rechtzeitig zu erkennen.

Vielfältige Versuche der intraoperativen Identifizierung des NLR und des intraoperativen Monitorings seiner Funktion sind bereits unternommen worden. Keines dieser Verfahren läßt jedoch eine kontinuierliche Überwachung des Nerven zu. So wurde von Dale B. Smith und anderen in dem Aufsatz "A device for the intraoperative identification of the recurrent laryngeal nerve in piglets" (Otolaryngology - Head and Neck Surgery 1989, 100, S. 137-145) der eingangs angegebene Mehrballon-Endotrachealtubus beschrieben. Ein entsprechender Endotrachealtubus geht aus der NL-A-7908514 als bekannt hervor. Die dem oberen Ballon zugeordneten Sensoren arbeiten dabei hydraulisch. Der entsprechende Tubus wird dabei so weit in die Luftröhre eingeführt, daß der obere Ballon im Bereich der Stimmritze zu liegen kommt. In dieser Stellung wird der Tubus in der Luftröhre fixiert und gegenüber dieser abgedichtet, indem der untere Ballon aufgepumpt wird. Anschließend wird der obere Ballon so weit expandiert, bis er an den Stimmbändern anliegt. Der NLR wird intraoperativ elektrisch stimuliert, was unter der Voraussetzung, daß der NLR nicht geschädigt ist, zu einer entsprechenden Muskelkontraktion der Stimmritze führt. Diese Muskelkontraktion führt zu einer Druckerhöhung in dem oberen Ballon, welche auf einem Manometer, an das dieser angeschlossen ist, angezeigt wird.

Der entscheidende Nachteil dieses bekannten Endotrachealtubus liegt darin, daß sich mit ihm nur beträchtliche bereits vollzogene Schädigungen des NLR nachweisen lassen, die sich in groben Veränderungen seiner Funktion niederschlagen. Nicht nachweisbar ist hingegen die auf äußere Einflüsse zurückgehende Beeinträchtigung einzelner Axone des Nervs. Ebensowenig lassen sich mit dem bekannten Endotrachealtubus leichte und reversible Traumen des NLR wie bspw. Zug am Nerven und somit dessen drohende, noch nicht eingetretene Schädigung darstellen. Dieses System ist für eine kontinuierliche Überwachung nicht geeignet, da die Sensitivität zu gering und die Störanfälligkeit durch leichte Druckschwankungen zu gravierend ist.

Da der NLR nicht nur zu einer Schließung, sondern auch zu einer Öffnung der Stimmbänder dient, könnten Traumen unentdeckt oder auch fehlinterpretiert werden, wenn diese nicht zu einer Druckerhöhung, sondern zu einer Druckkonstanz oder auch Drucksenkung führen.

Nicht erfaßbar sind mit dem Verfahren die durch mechanische Berührung ausgelösten elektrischen Eigenaktivitäten des Nerven, die als sogenannte "spikes" im EMG in Erscheinung treten. Deren Dauer ist zu kurz, um durch das grobe Verfahren der Druckmessung erkannt und quantifiziert zu werden. Grundsätzlich löst damit eine Druckmessung in der Stimmritze nicht die zu lösende Aufgabe eines zuverlässigen intraoperativen, kontinuierlichen Monitoring der Kehlkopfnerven.

Darüber hinaus wurden verschiedene andere Verfahren und entsprechende Geräte beschrieben, die zur intraoperativen Darstellung des elektrisch stimulierten NLR vorgesehen waren. D.J. Premachandra und andere beschrieben in ihrem Aufsatz "Intraoperative identification of the recurrent laryngeal nerve and demonstration of its function" (Laryngoscope 1990, 100, S. 95-96) die Visualisierung der Stimmbandbeweglichkeit mittels eines starren Endoskops. A.G. James und andere legten in ihrem Aufsatz "A simple method for identifying and testing the recurrent laryngeal nerve" (Surgery Gynecology Obstetrics 1985, 161; S. 185-186) dar, daß die Integrität des NLR durch Palpation des M. cricothyroideus getestet werden kann. J.G. Spahn und andere beschrieben in ihrem Aufsatz "Identification of the motor laryngeal nerves - a new electrical stimulation technique" (Laryngoscope 1991, 91; S. 865-868), daß eine feine in der Stimmfalte plazierte Nadel bei einer durch elektrische Stimulation des NLR ausgelösten Bewegung der Stimmritze sichtbare Bewegungen ausführt. Ein traumatisches Ableitverfahren mittels einer bipolaren Nadelelektrode wurde von W.E. Davis in seinem Aufsatz "Recurrent laryngeal nerve localisation using a microlaryngeal electrode" (Otolaryngology - Head and Neck Surgery, Vol. 87, S. 330 ff.) beschrieben. Jedoch konnte sich wegen technischer Schwierigkeiten und/oder traumatisierender Ableitverfahren keine dieser Methoden durchsetzen. Das zuletzt genannte Verfahren weist dabei zudem den Nachteil auf, daß nur einzelne motorische Einheiten ableitbar sind; über die Integrität des gesamten Nervs sagt das Verfahren daher nichts aus. Die Ableitung mittels wie immer gearteter Nadel- oder Hakenelektroden kann das Problem eines intraoperativen Monitoring der Kehlkopfnerven nicht zuverlässig lösen. Ein kontinuierliches Monitoring-Verfahren existiert nicht.

J. Lee Rea beschreibt schließlich in seinem Aufsatz "Postcricoid Surface Laryngeal Electrode" (Ear, Nose and Throat Journal, Vol. 71, Nr. 6, S. 267 ff.) die atraumatische Ableitung des NLR mittels eines mit Oberflächenelektroden versehenen Körpers, der in die Stimmritze eingeführt werden soll. Die hier beschriebene Vorrichtung ist jedoch praktisch für den vorgesehenen Zweck nicht brauchbar. Denn ihr Einsatz kann eine Schädigung des umgebenden Gewebes nach sich ziehen. Darüber hinaus läßt sich die Vorrichtung nicht mit ausreichender Sicherheit an der vorgesehenen Stelle plazieren, und eine Dislocation während ihres Einsatzes ist nicht sicher ausgeschlossen.

Die Dislokation der Elektroden und die Ungenauigkeit der Ableitung schon bei kleinen Bewegungen ist das Hauptproblem des Endotrachealtubus, der von Andrew Goldstone beschrieben wurde (EP-0438863 A1). Bei diesem Tubus sind exakt parallel der Tubusachse zwei Drähte am Tubus angebracht, die die elektrische Aktivität der Stimmbänder erfassen sollen. Eine sichere Ableitung ist dadurch jedoch nicht möglich, da schon bei leichter Bewegung oder Drehung des Tubus der Kontakt zu den Stimmbändern aufgehoben werden kann. Auch der unterschiedliche Öffnungszustand der Stimmbänder kann eine Ableitungsstörung hervorrufen. Der hierbei veränderte Anpreßdruck kann bei noch erhaltener Signalableitung zu Amplitudenveränderungen des Signales führen. Die Erkennung einer drohenden Läsion des NLR und des NLS kann daher mit diesem Gerät grundsätzlich nicht erfolgen. Ein kontinuierliches Monitoring des NLS und des NLR ist damit nicht praktikabel. Da immer ein maximal dicker Tubus benötigt wird, um den Kontakt zu den Stimmbändern herzustellen, steigt die Verletzungsgefahr für die Stimmbänder dramatisch an, da der Beatmungsschlauch mit den längsgestellten Oberflächendrähten durch die Stimmbänder gezwängt werden muß. Ein Einschneiden in die Stimmbänder wird dadurch denkbar. Die von Goldstone beschriebene Vorrichtung ist also für den vorgesehen Zweck nicht brauchbar.

Der vorliegenden Erfindung liegt die Aufgabe zugrunde, einen Mehrballon-Endotrachealtubus der eingangs genannten Art zu schaffen, der ein ausreichend sensibles und sicher zu positionierendes Detektions- und Überwachungssystem darstellt, das - unter Verwendung geeigneter Auswertegeräte - ermöglicht, die motorischen Stimmbandnerven (NLR und NLS) in ihrem Verlauf im Gewebe zu detektieren und intraoperativ in ihrer Funktion kontinuierlich zu überwachen derart, daß selbst leichte und reversible Läsionen unter Einschluß von Beeinträchtigungen nur weniger Axone der Nerven erkannt werden, um bereits eine drohende Schädigung anzuzeigen, wobei ein kontinuierliches Monitoring außerhalb des OP-Feldes möglich wird, was den technischen Ablauf der Operation erstmals nicht mehr beeinträchtigt.

Gemäß der vorliegenden Erfindung wird diese Aufgabe dadurch gelöst, daß auf der Oberfläche des oberen Ballons elektrische und/oder elektromagnetische Sensoren angeordnet sind und daß am unteren Ballon mindestens eine zusätzliche Stimulationselektrode zur transtrachealen oder transbronchialen Stimulation des Nervus laryngeus recurrens angeordnet ist. Die am oberen Ballon angeordneten Sensoren detektieren die elektrische oder elektromagnetische Aktivität der Kehlkopfmuskeln insbesondere als Folge der elektrischen Reizung der Kehlkopfnerven mittels der am unteren Ballon vorgesehenen Stimulationselektrode(n). Sie zeigen somit insbesondere eine als Reaktion auf die elektrische Stimulation des NLR und/oder des NLS auftretende Änderung des physikalischen Zustands der an ihnen anliegenden Stimmbänder an. Die vorliegende Erfindung macht sich somit die Erkenntnis zunutze, daß sich bei fortgesetzter elektrischer Stimulation des NLR der physikalische Zustand der Stimmbänder bereits bei einer derartig geringfügigen mechanischen Beeinträchtigung des NLR verändert, bei der eine ableitbare Bewegungsänderung der Stimmbänder noch nicht auftritt. Dies führt dazu, daß, verglichen mit den bekannten Verfahren, die eine Bewegung der Stimmbänder ableiten, unter Anwendung des erfindungsgemäßen Endotrachealtubus bereits eine deutliche geringfügigere mechanische Beeinträchtigung des NLR nachweisbar ist. Die auf dem unteren Ballon angeordnete(n), zur transtrachealen Stimulation des NLR geeignete(n) Stimulationselektrode(n) macht/machen sich eine als solches bekannte Stimulationstechnik zunutze, die von J.A. Sercarz u.a. in "Fabrication of a custom electrode endotracheal tube" (Laryngoscope 1991, 101; S. 1024-1025) vorgeschlagen wurde. Die durch den erfindungsgemäßen Mehrballon-Endotrachealtubus mögliche transtracheale Stimulation des NLR in Verbindung mit der weiter oben erläuterten hochsensiblen Ableitung läßt erstmals eine kontinuierliche intraoperative Überwachung zu, ohne daß der Operateur sich in irgendeiner Weise weiterhin darum kümmern muß. Die besondere Bedeutung des erfindungsgemäßen Endotrachealtubus ist darin zu sehen, daß sich die Positionierung der Stimulationselektroden und der Sensoren aus der Routine-Intubation des Patienten ergibt und gleichzeitig damit auch abgeschlossen ist und somit keine zusätzlichen Manipulationen erforderlich sind. Darüberhinaus ist die gesamte Vorrichtung völlig atraumatisch für den Pätienten.

Die gesamte Apparatur liegt damit außerhalb des OP-Feldes und erlaubt ein atraumatisches Monitoring des NLR schon vor dem Hautschnitt bis zum Ende der Narkose.

Zweckmäßigerweise wird beim Einsatz des erfindungsgemäßen Tubus zunächst der NLR unter Verwendung einer bipolaren Stimulationselektrode im Operationsgebiet aufgespürt und anschließend auf die transtracheale Stimulation umgeschaltet, so daß der Operateur fortan beide Hände frei hat. Die Operation kann somit erstmals unter kontinuierlicher Überwachung der Nervenfunktion erfolgen, ohne daß der operationstechnische Ablauf geändert werden müßte.

Ein Einzelballontubus, dessen Elektroden an einen Elektrokardiographen oder Defibrillator anschließbar sein sollen, ist zwar bekannt (DE-GM 8915538), zeigt aber keine Verbindung zu dem Problem, das zu lösen die Aufgabe der vorliegenden Erfindung ist.

Bei einer besonders bevorzugten Weiterbildung des erfindungsgemäßen Endotrachealtubus sind die auf dem oberen Ballon angeordneten Sensoren als elektrisch leitende, insbesondere flächige Streifenelektroden ausgebildet. Mit ihnen läßt sich eine Änderung des elektrischen Potentials der an den Streifenelektroden anliegenden Stimmbänder ermitteln. In diesem Falle ist die physikalische Größe der Stimmbänder, welche abgeleitet wird, somit deren elektrisches Summen-Potential. Einsetzbar sind im Rahmen der vorliegenden Erfindung jedoch bspw. auch lineare Elektroden. Zweckmäßigerweise sind zwei Elektroden vorgesehen. Jedoch ist für einzelne Anwendungen auch eine andere Anzahl von Elektroden oder anders geartete elektromagnetische Sensoren mit Vorteil einsetzbar.

Eine bevorzugte Weiterbildung des erfindungsgemäßen Endotrachealtubus zeichnet sich dadurch aus, daß die Sensoren als elektrisch leitende Oberflächenbeschichtung des oberen Ballons ausgebildet sind.

Bei einer Weiterbildung des erfindungsgemäßen Endotrachealtubus sind die Elektroden am oberen Ballon als kombinierte Stimulations-/Ableitelektroden ausgebildet oder zusätzliche Stimulationselektroden angebracht. Auch ist ein anders gearteter elektromagnetischer Stimulator denkbar. Durch diese Anordnung wird erreicht, daß die sensorischen Äste des NLS erstmals über die Schleimhaut des Kehlkopfes stimuliert werden können und über den bekannten Reflexbogen *) im vagalen Nucleus auf den motorischen Ast des NLS umgeschaltet werden. Mit einer Latenz von ca. 5ms erreicht das Reflexbogensignal ("H-Wave") den M. cricothyroideus. Das Summenaktionspotential ist dann über die Sensoren am oberen Ballon ableitbar. Die Integrität des gesamten NLS (einschl. sensorischer und motorischer Fasern) kann damit erstmals kontinuierlich während der Operation (ohne Tangierung des OP-Feldes) überwacht werden. Diese Art der NLS-Stimulierung mit Ausnutzung des Reflexbogens ist von besonderer Bedeutung, da dadurch erstmals ein kontinuierliches Monitoring des NLS ohne Tangierung des OP-Feldes möglich wird.
*) W.F. Thumfart "Electromyography of the larynx and related technics". Acta Oto Rhino Laryngologica Belgica 1986, 40:2, S. 358 ff.

Für einen anderen Anwendungsbereich (Messung der Narkosetiefe bzw. der Relaxationstiefe bei sonstigen Operationen) ist der erfindungsgemäße Endotrachealtubus mit der bereits bekannten hydraulischen Druckmessung über den oberen Ballon kombiniert. Dies ermöglicht eine gleichzeitige Erfassung der mechanischen Aktivität in der Stimmritze. Diese dient zur exakten Steuerung der Narkosetiefe bzw. der Relaxationstiefe des Patienten, erkennbar am Verlust der motorischen Aktivität (muskuläre Lähmung) und Erhalt einer elektrischen bzw. elektromagnetischen Restaktivität der Muskulatur. Der Vorteil gegenüber der bisher üblichen Prüfung der Relaxationstiefe des Patienten durch Stimulation der Handnerven und der Beobachtung der daraus folgenden Kontraktion der Handmuskeln ist eine exakte und reproduzierbare Muskelkraftmessung zusätzlich zum EMG-Signal und damit eine bessere Steuerung der Narkose bzw. Relaxationstiefe durch den erfindungsgemäßen Endotrachealtubus. Die Stimmbandbeweglichkeit ist bekanntermaßen abhängig von der Relaxationstiefe des Patentien (siehe den Aufsatz von J.O. Dich-Nielsen "Flexible fiberoptic bronchoscopy via the laryngeal mask"; (Acta Anaesthesiologica scandinavica 1993, 37; S. 17 ff.).

Die Erfindung wird nachstehend unter Bezugnahme auf die Zeichnung näher erläutert. Dabei zeigt
- Fig. 1: einen Sagitalschnitt durch ein im Kehlkopf plaziertes bevorzugtes Ausführungsbeispiel des erfindungsgemäßen Endotrachealtubus,
- Fig. 2: einen Frontalschnitt durch den im Kehlkopf plazierten Endotrachealtubus gemäß Fig. 1,
- Fig. 3: bis 8 verschiedene typische abgeleitete Spannungsverläufe bei unbeeinträchtigtem und auf verschiedene Weise mechanisch beeinträchtigtem NLR,
- Fig. 9: einen typischen Spannungsverlauf nach transtrachealer Stimulation über eine Stimulationselektrode am unteren Ballon ohne und mit traumatischer Schädigung des NLR und
- Fig. 10: einen typischen Spannungsverlauf nach Stimulation des NLS ohne traumatische Schädigung.

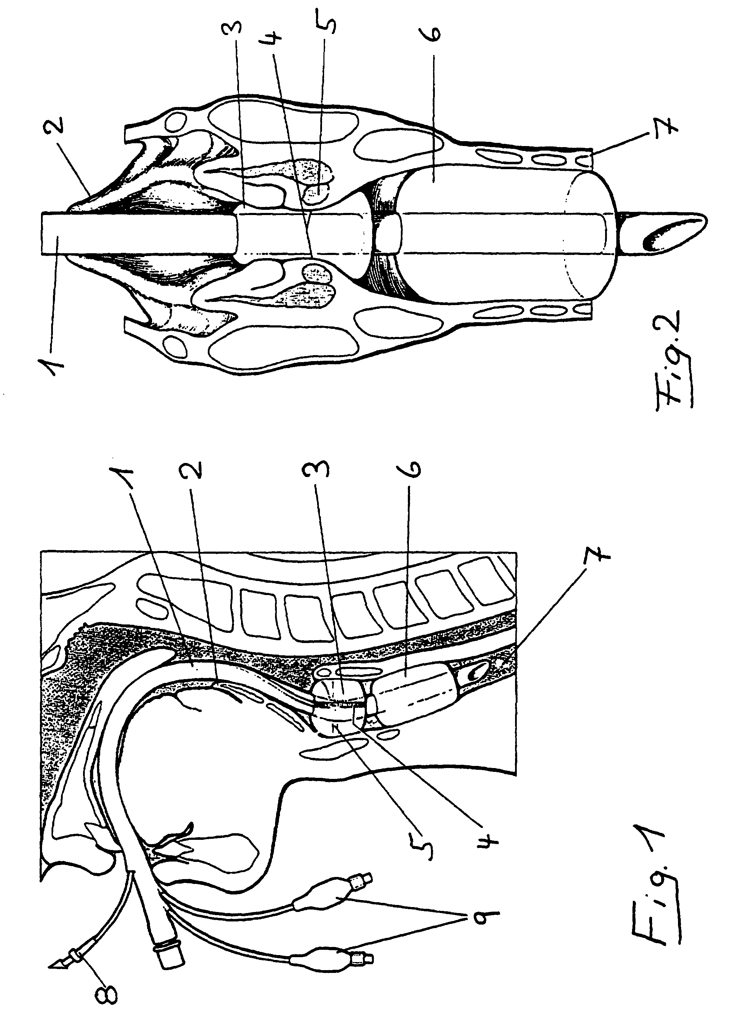

Der Endotrachealtubus gemäß den Fig. 1 und 2 umfaßt einen Schlauchabschnitt 1. Benachbart dem unteren Ende des Tubus sind zwei Ballons 3 bzw. 6 angeordnet, welche mittels der beiden Pumpmechaniken 9 über entsprechende Verbindungsschläuche unabhängig voneinander expandiert werden können. Insoweit entspricht der in Fig. 1 dargestellte Endotrachealtubus dem im Handel erhältlichen Stand der Technik, so daß es keiner näheren Erläuterungen bedarf.

Auf der Oberfläche des oberen Ballons 3 sind zwei elektrisch leitende Streifenelektroden 4 angeordnet, von denen aufgrund ihrer Stellung zueinander in Fig. 1 nur die dem Betrachter zugewandte sichtbar ist. An jede der beiden Streifenelektroden 4 ist ein elektrisch leitendes Ableitkabel angeschlossen, das entlang dem Tubus in/an dessen Wandung fixiert sich zu dem unteren Ende erstreckt. An den Enden der beiden Ableitkabel sind Anschlußstecker 8 zu ihrem Anschluß an einer Auswerteelektronik vorgesehen.

Die Fig. 1 und 2 veranschaulichen die Plazierung des erfindungsgemäßen Endotrachealtubus in der Weise, daß der obere Ballon 3 in der Stimmritze zu liegen kommt. In Fig. 2 sind der Musculus vocalis und der Musculus thyroarytaenoideus 5 angedeutet; Fig. 1 zeigt bei 2 die Epiglottis. Mit 7 ist jeweils die Trachea bezeichnet. Gegen sie dichtet der untere Ballon 6 ab; zugleich sorgt er zuverlässig und sicher für eine Beibehaltung der korrekten Position des oberen Ballons 3 auch während länger andauernder Operationen. Die Anschmiegung der auf dem oberen Ballon 3 angeordneten Oerflächen-Ableitelektroden 4 an die Stimmbänder ist Fig. 2 gut entnehmbar.

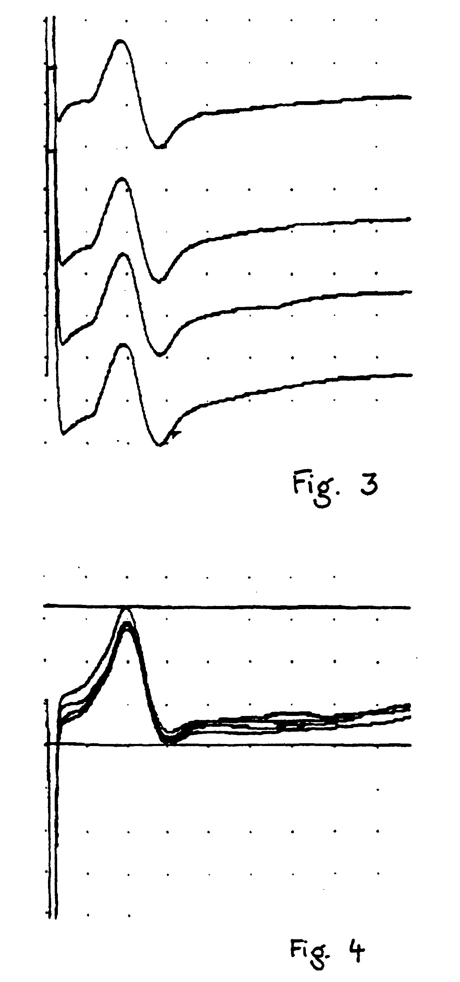

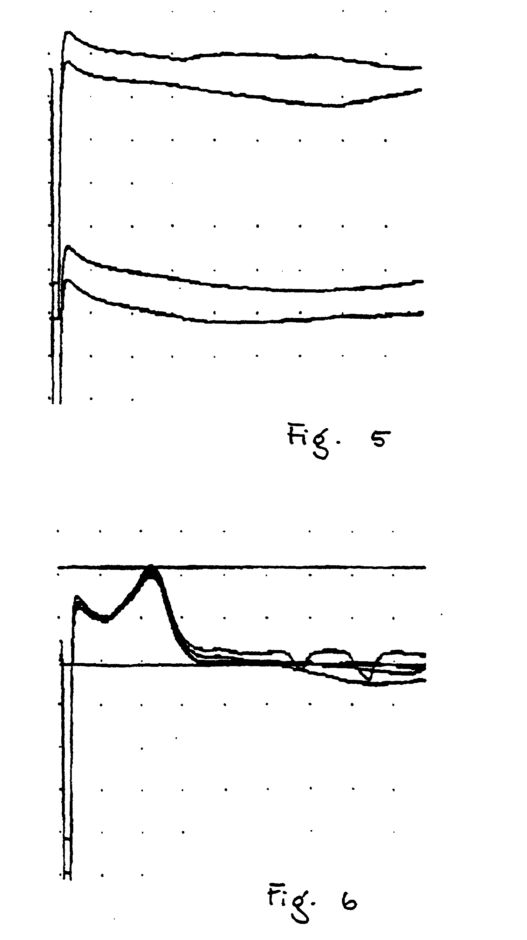

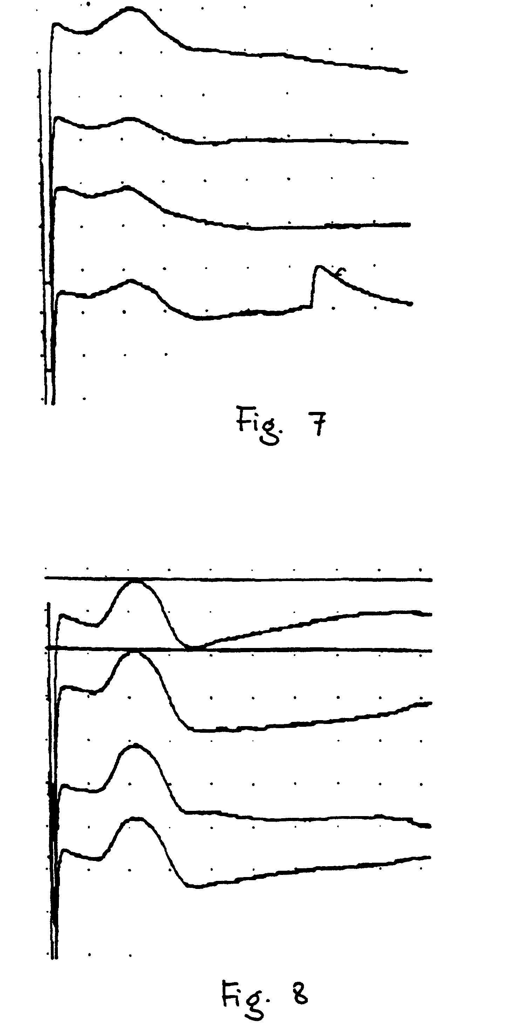

Die in den Fig. 3 bis 8 dargestellten abgeleiteten Spannungen wurden bei Versuchen aufgezeichnet, die unter Verwendung des Endotrachealtubus gemäß den Fig. 1 und 2 an jungen Schweinen durchgeführt wurden. Dabei wurde im einzelnen wie folgt vorgegangen: Als Stimulationselektroden wurden modifizierte konventionelle atraumatische bipolare Klammerelektroden eingesetzt. Der Elektrodenabstand betrug 1 cm. Als Erdungselektrode fand eine monopolare Drahtelektrode mit großflächiger Gegenelektrode Verwendung. Die Nervenstimulation erfolgte durch einen Konstant-Strom-Stimulator mit 10 - 20 mA.

Der Endotrachealtubus wurde (mit vollständig entleerten Ballons 3 und 6) in die Trachea eingeführt, und zwar so weit, bis der untere Ballon 6 mit Sicherheit unterhalb der Glottis lag. Anschließend wurde der untere Ballon 6 leicht aufgepumpt (geblockt). Sodann wurde der Endotrachealtubus so weit zurückgezogen, bis der partiell geblockte untere Ballon sich in der Subglottis verfing. In dieser Stellung des Tubus wurde sowohl der untere Ballon 6 als auch der obere Ballon 3 expandiert, so daß der untere Ballon 6 den Schlauchabschnitt 1 gegen die Trachea abdichtete und die auf den oberen Ballon 3 angeordneten Streifenelektroden 4 in direktem Kontakt an den Stimmbändern anlagen.

Die in den Fig. 3 bis 8 dargestellten Summenpotentiale der Stimmbandmuskeln (dem Erfolgsorgan des NLR) wurden über ein Standard-EMG-Gerät ("Dantec Cantata") abgeleitet. Dabei kam ein Bandpaßfilter 20 Hz bis 2 kHz zur Anwendung. Die Verstärkung betrug 1 mV/Raster. Die Ableitung erfolgte monopolar ipsilateral am Stimmband. Die abgeleiteten Potentiale waren in ihrer Form exakt reproduzierbar und erreichten eine Signalstärke von 2,2 bis 3,0 mV.

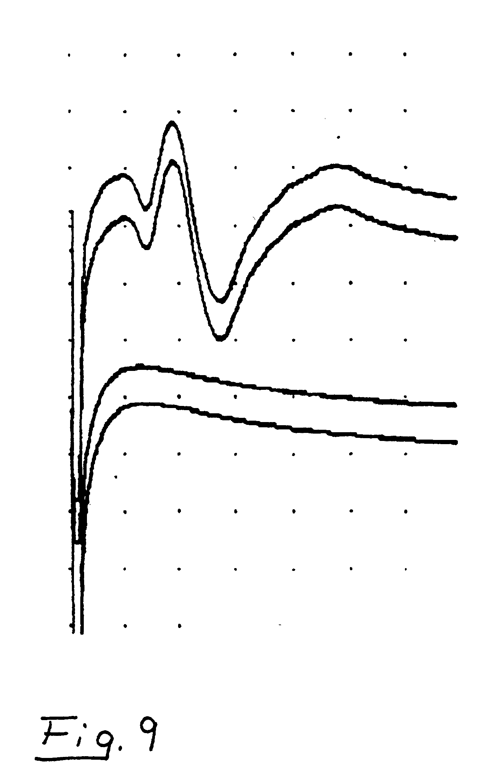

In Fig. 3 sind vier voneinander unabhängige Messungen im zeitlichen Abstand von 0,5 bis 4 min dargestellt, die eine typische Ableitung nach einer Stimulation mit 14,3 mA am peripheren Nerven bilden. Auf der Abszisse dargestellt ist der Zeitverlauf (2 msec pro Raster). Auf der Ordinate dargestellt ist die abgeleitete Spannung des Summen-Aktionspotentials (1 mV pro Raster). Fig. 3 veranschaulicht die gute Reproduzierbarkeit der abgeleiteten Potentiale. Zur Verdeutlichung der Reproduzierbarkeit wurden gemäß Fig. 4 vier weitere voneinander unabhängige Ableitungen übereinander projiziert. Der monomorphe Signalverlauf ist auf diese Weise gut erkennbar. Wie bereits bei Fig. 3 ist auch bei Fig. 4 auf der Abszisse der Zeitverlauf (2 msec pro Raster) und auf der Ordinate die abgeleitete Spannung des Summen-Aktionspotentials (1 mV pro Raster) dargestellt.

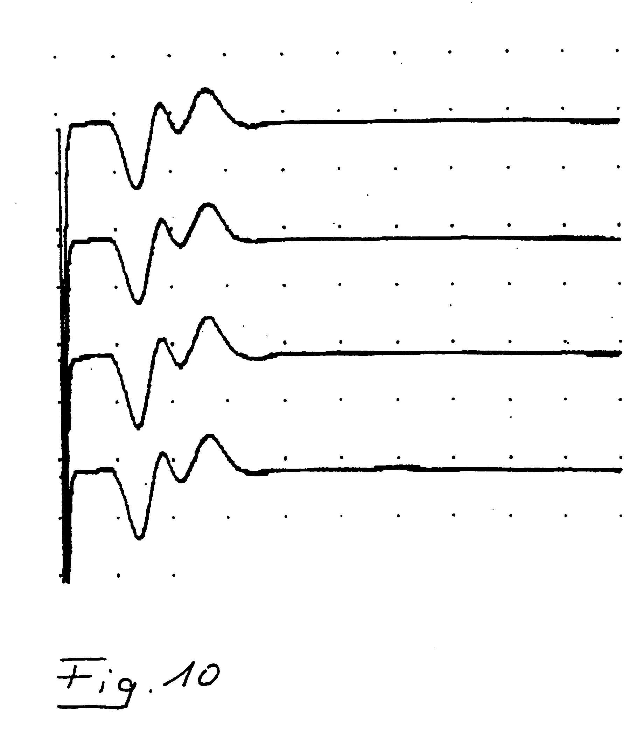

Fig. 5 zeigt das abgeleitete Summen-Aktionspotential unter den gleichen Bedingungen, nachdem der entsprechende NLR mit einer Pinzette komprimiert wurde. Erkennbar führt diese Form der mechanischen Beeinträchtigung des NLR zu einem kompletten Verlust der Aktionspotentiale.

Fig. 6 zeigt die abgeleiteten Summen-Aktionspotentiale, nachdem die Pinzette wieder geöffnet wurde und somit eine Dekompression des NLR eintrat. Ersichtlich führte die Dekompression des NLR zu einer fast vollständigen Wiederherstellung der Nervenleitfähigkeit. Die in Fig. 5 dokumentierte Nervenleitstörung war somit fast völlig reversibel. Die leichte Verbreiterung und Verminderung des Aktionspotentials zeigt jedoch eine leichte, stattgehabte Schädigung des Nerven als Folge der starken Kompression an.

Von besonderer Bedeutung sind die in den Fig. 7 und 8 dargestellten Verläufe der Summen-Aktionspotentiale bei zwei vergleichsweise leichten Traumen am Nerven. Und zwar gibt Fig. 7 - unter den im übrigen übereinstimmenden Bedingungen wie die Fig. 3 bis 6 - die Summen-Aktionspotentiale bei einer sehr leichten Kompression des NLR wieder. Ersichtlich führte diese (verglichen mit den Fig. 5 zugrundeliegenden Verhältnissen) leichtere Kompression zu einem partiellen Verlust des Aktionspotentials. Dies dokumentiert, daß bereits eine relativ geringe Kompression des Nerven, welche keinerlei bleibende Schäden hinterläßt, unter Verwendung des erfindungsgemäßen Tubus nachweisbar ist.

Dasselbe gilt für einen Zug am Nerven, dessen Auswirkungen in Fig. 8 veranschaulicht sind. Bereits ein geringfügiger Zug am Nerven führt zu einer deutlich erkennbaren Verplumpung des Signalsverlaufs, wie ein Vergleich mit Fig. 3, welche den Signalverlauf bei unbeeinträchtigtem NLR wiedergibt, deutlich zeigt.

Fig. 9 veranschaulicht das exzellent auswertbare Signal, das sich bei transtrachealer Stimulation des NLR mittels an dem unteren Ballon vorgesehener Stimulationselektroden ergibt. Die beiden oberen Kurven geben typische Spannungsverläufe ohne·Schädigung des NLR wider; die unteren beiden Kurven wurden bei einer traumatischen Schädigung des NLR aufgezeichnet. Der erfindungsgemäße Endotrachealtubus ermöglicht nun erstmals ein kontinuierliches intraoperatives Monitoring des NLR ohne Beeinträchtigung des operativen Ablaufes durch Stimulation und Ableitung außerhalb des OP-Feldes.

Fig. 10 veranschaulicht das hervorragend reproduzierbare und auswertbare Summenaktionspotential, das sich unter Verwendung des erfindungsgemäßen Tubus als Reaktion auf eine Stimulierung des NLS aufzeichnen läßt. Damit konnte erstmals eine intraoperative EMG-Ableitung des Musculus cricothyroideus (Erfolgsorgan des NLS) außerhalb des OP-Feldes erfolgen.

In einer Weiterbildung des erfindungsgemäßen Endotrachealtubus erfolgt die Stimulierung des NLS außerhalb des OP-Feldes durch zusätzliche Stimulationselektroden am oberen Ballon, die auch identisch mit den Ableitsensoren sein können. Dabei kann ein bekannter neurophysiologischer Reflexbogen ausgenutzt werden, der es ermöglicht, den gesamten NLS einschließlich seiner sensorischen Fasern im gesamten Verlauf kontinuierlich zu überwachen.

## Patentansprüche

1. Mehrballon-Endotrachealtubus, umfassend einen Schlauchabschnitt (1) und mindestens zwei einander benachbart angeordnete, unabhängig voneinander expandierbare Ballons (3; 6), wobei einem ersten, oberen, oberhalb eines zweiten, unteren Ballons (6) angeordneten Ballon (3) atraumatische Sensoren zur Prüfung der motorischen Kehlkopfnerven zugeordnet sind,
dadurch gekennzeichnet,
daß auf der Oberfläche des oberen Ballons (3) elektrische und/oder elektromagnetische Sensoren (4) angeordnet sind und daß am unteren Ballon (6) mindestens eine zusätzliche Stimulationselektrode zur transtrachealen oder transbronchialen Stimulation des Nervus laryngeus recurrens angeordnet ist.

2. Endotrachealtubus nach Anspruch 1,
dadurch gekennzeichnet,
daß die Sensoren (4) als elektrisch leitende Oberflächenbeschichtung des oberen Ballons (3) ausgebildet sind.

3. Endotrachealtubus nach Anspruch 1,
dadurch gekennzeichnet,
daß die Sensoren am oberen Ballon als kombinierte Stimulations-/Ableitelektroden ausgebildet sind oder zusätzliche Stimulationselektroden oder andersgeartete Nervenstimulatoren angebracht sind.

4. Endotrachealtubus nach Anspruch 1,
dadurch gekennzeichnet,
daß der obere Ballon (3) zusätzlich als pneumatischer oder hydraulischer Drucksensor ausgelegt und an ein Druckmeßgerät angeschlossen ist.

## Claims

1. Multiple balloon endotracheal tube, comprising a hose section (1) and at least two adjacent, independently inflatable balloons (3,6), whereby atraumatic sensors for monitoring the motor laryngeal nerves are allocated to a first, upper balloon (3) above a second, lower balloon (6), characterised in that electrical and/or electromagnetic sensors (4) are arranged on the surface of the upper balloon (3) and that at least one additional stimulation electrode is arranged on the lower balloon for the trans-tracheal or trans-bronchial stimulation of the recurrent laryngeal nerve.

2. Endotracheal tube in accordance with Claim 1, characterised in that the sensors (4) are designed as an electrically conductive surface coating on the upper balloon (3).

3. Endotracheal tube in accordance with Claim 1, characterised in that the sensors on the upper balloon are designed as combined stimulation/reference electrodes or additional stimulation electrodes or nerve stimulators of another kind are attached.

4. Endotracheal tube in accordance with Claim 1, characterised in that the upper balloon (3) is additionally designed as a pneumatic or hydraulic pressure sensor and is connected to a pressure measuring device.

## Revendications

1. Tube endotrachéal à plusieurs ballonnets, comprenant un tronçon de tube souple (1) et au moins deux ballonnets (3, 6) qui sont disposés dans le voisinage l'un de l'autre et peuvent être gonflés indépendamment l'un de l'autre, des détecteurs atraumatiques,pour l'examen des nerfs moteurs du larynx, étant associés à un premier ballonnet (3) supérieur disposé au-dessus d'un second ballonnet (6) inférieur, caractérisé en ce que des détecteurs (4) électriques et/ou électromagnétiques sont disposés sur la surface du ballonnet (3) supérieur et en ce qu'au moins une électrode de stimulation supplémentaire, pour la stimulation transtrachéale ou trans-bronchiale du nervus laryngeus recurrens, est disposée sur le ballonnet (6) inférieur.

2. Tube endotrachéal selon la revendication 1, caractérisé en ce que les détecteurs (4) sont conformés en revêtement de surface électro-conducteur du ballonnet (3) supérieur.

3. Tube endotrachéal selon la revendication 1, caractérisé en ce que les détecteurs sur le ballonnet supérieur sont conformés en électrodes combinées de stimulation / prélèvement ou que des électrodes de stimulations supplémentaires ou des stimulateurs nerveux d'un type autre sont disposés sur le ballonnet supérieur.

4. Tube endotrachéal selon la revendication 1, caractérisé en ce que le ballonnet (3) supérieur est en outre conformé en détecteur de pression pneumatique ou hydraulique et est connecté à un appareil de de mesure de pression
